# EUROPEAN PATENT SPECIFICATION

(11) **EP 1 159 258 B1**
(45) Date of publication and mention of the grant of the patent: **10.11.2004**
(21) Application number: 00902080.1
(22) Date of filing: 03.02.2000
(51) Int. Cl.: C07C 233/65, C07C 275/30, C07D 211/68, C07D 217/06, C07D 213/56, A61K 31/44, A61K 31/16, A61P 25/28

(54) **AMIDE COMPOUNDS FOR THE POTENTIATION OF CHOLINERGIC ACTIVITY**
AMIDVERBINDUNGEN ZUR STÄRKUNG DER CHOLINERGISCHEN WIRKUNG
COMPOSES AMIDE POUR LA SYNERGIE DE L'ACTIVITE CHOLINERGIQUE

(30) Priority: 26.02.1999 AU PP891299
(43) Date of publication of application: 05.12.2001
(73) Proprietor: FUJISAWA PHARMACEUTICAL CO., LTD., Osaka-shi Osaka 541-8514 (JP)
(72) Inventor: Yamada, Akira, Osaka 583-0011 (JP); Aoki, Satoshi, Kanagawa 239-0801 (JP)
(74) Representative: Gille Hrabal Struck Neidlein Prop Roos
(86) International application number: PCT/JP2000/000601
(87) International publication number: WO 2000/051970

(56) References cited:
- EP-A- 0 306 375
- EP-A- 0 343 961
- FR-M- 8 287
- US-A- 4 797 419
- YEUNG, J.M. & KNAUS, E.E.: "Synthesis of 3,6-dihydro-1(2H)-pyridinyl derivatives with hyperglycemic activity" EUR. J. MED. CHEM. - CHIM- THER, vol. 21, no. 3, 1986, pages 181-185, XP000882437
- AGWADA, V.: "Potential Central Nervous System Active Agents. 3. Synthesis of Some Substituted Benzamides and Phenylacetamides" J. CHEM. ENG. DATA, vol. 29, no. 2, 1984, pages 231-235, XP000882455
- PATENT ABSTRACTS OF JAPAN vol. 018, no. 385 (C-1227), 20 July 1994 (1994-07-20) & JP 06 107544 A (TAISHO PHARMACEUT CO LTD), 19 April 1994 (1994-04-19)
- PATENT ABSTRACTS OF JAPAN vol. 1995, no. 01, 28 February 1995 (1995-02-28) & JP 06 298732 A (TAISHO PHARMACEUT CO LTD), 25 October 1994 (1994-10-25)

## Description

### TECHNICAL FIELD

This invention relates to amide compounds and salts thereof which are useful as a medicament.

### BACKGROUND ART

Some aminopiperazine derivatives have been known as useful anti-amnesia or anti-dementia agents, for example, in PCT International Publication Nos. WO 91/01979 and WO 98/35951.

Disubstituted or trisubstituted urea derivatives with cholinergic and cholinomimetic activity are described in US 4,797,419. One such compound is N-(3-chloro-2-methylphenyl)-4-methyl-1-piperidine.

Further compounds with central nervous activity are described by Agwada (J. Chem. Eng. Data 1984; 29: 231-235) and are disclosed in EP -A- 0 343 961, JP -A- 06 107544 (Patent Abstracts of Japan Vol. 18, no. 385) and JP -A- 06 298732 (Patent Abstracts of Japan vol. 1995, no. 1).

### DISCLOSURE OF INVENTION

This invention relates to amide compounds and salts thereof.

More particularly, it relates to amide compounds and salts thereof which have the potentiation of the cholinergic activity, to processes for the preparation thereof, to a pharmaceutical composition comprising the same, and to a use for a manufacture of a medicament for the treatment and/or prevention of disorders in the central nervous system for mammals, and more particularly to method for the treatment and/or prevention of amnesia, dementia (e.g. senile dementia, Alzheimer's dementia, dementia associated with various diseases such as cerebral vascular dementia, cerebral post-traumatic dementia, dementia due to brain tumor, dementia due to chronic subdural hematoma, dementia due to normal pressure hydrocephalus, post-meningitis dementia, Parkinson's disease type dementia, etc.). Additionally, the object compound is expected to be useful as therapeutical and/or preventive agents for schizophrenia, depression, stroke, head injury, nicotine withdrawal, spinal cord injury, anxiety, pollakiuria, incontinence of urine, myotonic dystrophy, attention deficit hyperactivity disorder, excessive daytime sleepiness (narcolepsy), Parkinson's disease or autism.

One object of this invention is to provide new and useful amide compounds and salts thereof which possess the potentiation of the cholinergic activity.

Another object of this invention is to provide processes for preparation of the amide compounds and salts thereof.

A further object of this invention is to provide a pharmaceutical composition comprising, as an active ingredient, said amide compounds and salt thereof.

Still further object of this invention is to provide a use for the manufacture of a medicament for the treatment and/or prevention of aforesaid diseases in mammals, using the amide compounds and salts thereof.

The amide compounds of this invention can be represented by the following general formula [I]: 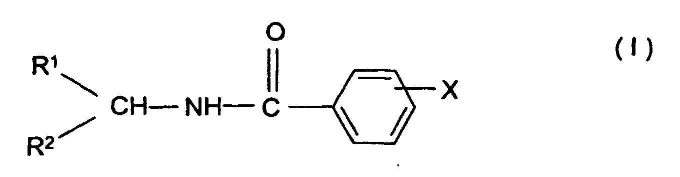 wherein R¹ and R² are taken together to form pentenylene condensed with benzene optionally substituted with (C₁-C₆) alkyl, (C₁-C₆) alkoxy, phenyl or halogen, and
X is halogen,
or,
R¹ and R² are taken together to form butenylene condensed with benzene, and
X is halogen,
and the indan ring formed by taking together R¹, R² and CH is substituted by -NH-CO-(substituted) phenyl at the 2-position,
and its salt.

The object compound [I] or its salt can be prepared by a process as illustrated in the following reaction scheme, reacting a compound of the formula: 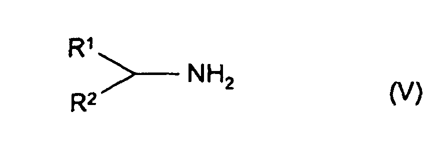 or its salt with a compound of the formula: 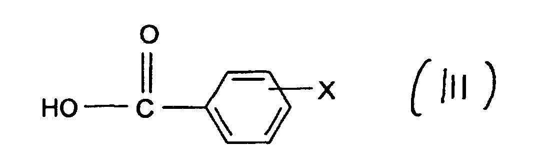 or its reactive derivative at the carboxy group or a salt thereof to provide a compound of the formula: 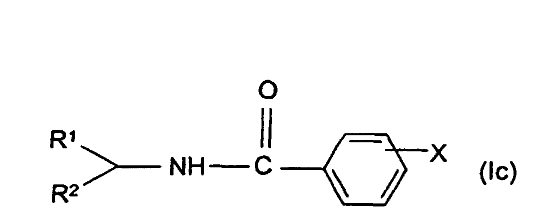 or its salt,
in the above formulas R¹, R² and X are each as defined above.

In the above and subsequent description of the present specification, suitable examples of the various definitions to be included within the scope of the invention are explained in detail in the following.

The term "lower" is intended to mean a group having 1 to 6 carbon atom(s), unless otherwise provided.

Suitable "lower alkyl" and lower alkyl moiety in the term "ar(lower)alkyl" may be a straight or branched C₁-C₆ alkyl such as methyl, ethyl, propyl, isopropyl, butyl, isobutyl, tert-butyl, pentyl, ethylpropyl, hexyl or the like, in which preferable one is methyl.

Suitable "aryl" and aryl or ar moiety in the terms "ar(lower)alkyl", "aryloxy" and "arylamino" may be phenyl, naphthyl, pentyl substituted with lower alkyl [e.g. tolyl, xylyl, mesityl, cumenyl, di(tert-butyl)phenyl, etc.] and the like, in which preferable one is phenyl.

Suitable "halogen" may be fluorine, chlorine, bromine and iodine, in which preferable one is fluorine.

Suitable "lower alkoxy" may be a straight or branched C₁-C₆ alkoxy such as methoxy, ethoxy, propoxy, isopropoxy, methylpropoxy, butoxy, isobutoxy, tert-butoxy, pentyloxy, hexyloxy or the like, in which preferable one is methoxy.

Suitable salts of the object compound [I] are pharmaceutically acceptable conventional non-toxic salts and include acid addition salt such as an inorganic acid addition salt [e.g. hydrochloride, hydrobromide, sulfate, phosphate, etc.], an organic acid addition salt [e.g. formate, acetate, trifluoroacetate, maleate, tartrate, methanesulfonate, benzenesulfonate, toluenesulfonate, etc.], a salt with an amino acid [e.g. aspartic acid salt, glutamic acid salt, etc.], a metal salt such as an alkali metal salt [e.g. sodium salt, potassium salt, etc.] and alkaline earth metal salt [e.g. calcium salt, magnesium salt, etc.] and the like.

The process for preparing the object compound [I] is explained in detail in the following.

### Process

The compound [Ic] or its salt can be prepared by reacting a compound [V] or its salt with a compound [III] or its reactive derivative at the carboxy group or a salt thereof.

Suitable salts of the compounds [Ic] and [V] may be the same as those exemplified for the compound [I].

Suitable salts of the compound [III] and its reactive derivative at the carboxy group may be metal salt or alkaline earth metal salt as exemplified for the compound [I].

Suitable reactive derivative at the carboxy group or the compound [III] may include an ester, an acid halide, an acid anhydride and the like. The suitable examples of the reactive derivatives may be an acid halide [e.g. acid chloride, acid bromide, etc.];
a symmetrical acid anhydride; a mixed acid anhydride with an acid such as aliphatic carboxylic acid [e.g. acetic acid, pivalic acid, etc.], substituted phosphoric acid [e.g. dialkylphosphoric acid, diphenylphosphoric acid, etc.];
an ester such as substituted or unsubstituted lower alkyl ester [e.g. methyl ester, ethyl ester, propyl ester, hexyl ester, trichloromethyl ester, etc.], substituted or unsubstituted ar(lower)alkyl ester [e.g. benzyl ester, benzhydryl ester, p-chlorobenzyl ester, etc.], substituted or unsubstituted aryl ester [e.g. phenyl ester, tolyl ester, 4-nitrophenyl ester, 2,4-dinitrophenyl ester, pentachlorophenyl ester, naphthyl ester, etc.], or an ester with N,N-dimethylhydroxylamine, N-hydroxysuccinimide, N-hydroxyphthalimide or 1-hydroxybenzotriazole, 1-hydroxy-6-chloro-1H-benzotriazole, or the like. These reactive derivatives can be optionally selected according to the kind of the compound [III] to be used.

The reaction is usually carried out in a conventional solvent such as water, acetone, dioxane, chloroform, methylene chloride, ethylene dichloride, tetrahydrofuran, ethyl acetate, N,N-dimethylformamide, pyridine or any other organic solvent which does not adversely influence the reaction. Among these solvents, hydrophilic solvent may be used in a mixture with water.

The reaction is also preferably carried out in the presence of a conventional base such as triethylamine, diisopropylethylamine, pyridine, N,N-dimethylaminopyridine, etc., or a mixture thereof.

When the compound [III] is used in a free acid form or its salt form in the reaction, the reaction is preferably carried out in the presence of a conventional condensing agent such as N,N'-dicyclohexylcarbodiimide, N-cyclohexyl-N'-morpholinoethylcarbodiimide, N-ethyl-N'-(3-dimethylaminopropyl)carbodiimide, thionyl chloride, oxalyl chloride, lower alkoxycarbonyl halide [e.g. ethyl chloroformate, isobutyl chloroformate, etc.], 1-(p-chlorobenzenesulfonyloxy)-6-chloro-1H-benzotriazole, or the like.

The reaction temperature is not critical, and the reaction can be carried out under cooling to heating.

The compounds obtained by the above processes can be isolated and purified by a conventional method such as pulverization, recrystallization, column chromatography, reprecipitation, or the like.

It is to be noted that the compound [I] and the other compounds may include one or more stereoisomer(s) such as optical isomer(s) or geometrical isomer(s) due to asymmetric carbon atom(s) and double bond(s), and all of such isomers and mixture thereof are included within the scope of this invention.

Additionally, it is to be noted that any solvate [e.g. enclosure compound (e.g. hydrate, ethanolate, etc.)] of the compound [I] or a salt thereof is also included within the scope of this invention.

The object compound [I] and salts thereof possess strong potentiation of the cholinergic activity, and are useful for the treatment and/or prevention of disorders in the central nervous system for mammals, and more particularly of amnesia, dementia (e.g. senile dementia, Alzheimer's dementia, dementia associated with various diseases such as cerebral vascular dementia, cerebral post-traumatic dementia, dementia due to brain tumor, dementia due to chronic subdural hematoma, dementia due to normal pressure hydrocephalus, post-meningitis dementia, Parkinson's disease type dementia, etc.) and the like. Additionally, the object compound is expected to be useful as therapeutical and/or preventive agents for schizophrenia, depression, stroke, head injury, nicotine withdrawal, spinal cord injury, anxiety, pollakiuria, incontinence of urine, myotonic dystrophy, attention deficit hyperactivity disorder, excessive daytime sleepiness (narcolepsy), Parkinson's disease or autism.

In order to illustrate the usefulness of the object compound [I], the pharmacological data of the compound [I] is shown in the following.

### Test

### Penile erection in rat

(This test was carried out according to a similar manner to that described in Jpn. J. Pharmacol., Vol. 64, 147-153 (1994))

### (i) Method

Male Fischer 344 rats at the age of 8 weeks (n=7) were used. All rats were handled 3 minutes a day for three successive days before the tests. The rats were tested in groups of seven and various doses of the test compound were given in semi-randomized order. The test compounds were suspended in 0.5% methyl-cellulose immediately before use, and given intraperitoneally in a volume of 1 ml/kg just before the start of test. Immediately after injection, each rat was placed in a perspex box (25x25x35 cm) and its behavior was observed for 60 minutes, during which time the number of penile erections was counted. A mirror was situated behind each box to observe the rat. Data was expressed as a mean number.

### (ii) Test Result

| Test Compound (Example No.) | Dose (mg/kg) | Penile Erection (Number/hr) |
|---|---|---|
| 2 | 0.32 | 0.57 |
| 6 | 0.32 | 0.60 |
| 8 | 0.1 | 0.60 |
| 7 | 0.1 | 0.71 |

It is clear that the compound having the above-mentioned activity ameliorates the memory deficits (i.e. amnesia, dementia, etc.) from the description in the Journal of Pharmacology and Experimental Therapeutics, Vo. 279, No. 3, 1157-1173 (1996). Further, it is expected that the compound having the above-mentioned activity is useful as therapeutical and/or preventive agent for aforesaid diseases from some patent applications (e.g. PCT International Publication No. WO 98/27930, etc.).

For therapeutic purpose, the compound [I] and a pharmaceutically acceptable salt thereof of the present invention can be used in a form of pharmaceutical preparation containing one of said compounds, as an active ingredient, in admixture with a pharmaceutically acceptable carrier such as an organic or inorganic solid, semi-solid or liquid excipient suitable for oral or parenteral administration. The pharmaceutical preparations may be capsules, tablets, dragees, granules, suppositories, solution, suspension, emulsion, or the like. If desired, there may be included in these preparations, auxiliary substances, stabilizing agents, wetting or emulsifying agents, buffers and other commonly used additives.

While the dosage of the compound [I] will vary depending upon the age and condition of the patient, an average single dose of about 0.1 mg, 1 mg, 10 mg, 50 mg, 100 mg, 250 mg, 500 mg and 1000 mg of the compound [I] may be effective for treating the above-mentioned diseases. In general, amounts between 0.1 mg/body and about 1,000 mg/body may be administered per day.

The following Preparations and Examples are given for the purpose of illustrating this invention.

### Preparation 1

To a solution of 4-methylcyclohex-3-enecarbonyl chloride (2 ml) in a mixture of methanol (20 ml) and tetrahydrofuran (20 ml) was added aqueous sodium hydroxide (4N, 20 ml). The resultant mixture was stirred at ambient temperature for 1 hour, and evaporated. The residue was taken up into a mixture of water and ethyl acetate and adjusted pH to around 1. The organic layer was separated, washed with brine, dried over magnesium sulfate, and evaporated under reduced pressure to give 4-methylcyclohex-3-enecarboxylic acid, which was used without further purification.
- NMR: (DMSO-d₆, δ) : 1.60 (3H, s), 1.35-1.65 (1H, m), 1.75-2.2 (5H, m), 2.25-2.45 (1H, m), 5.25-5.4 (1H, m), 12.09 (1H, br s)
- MASS: (LD)(m/z) : 139.2

### Preparation 2

To a solution of 4-methylcyclohex-3-enecarboxylic acid (1.7 g) and triethylamine (1.8 ml) in tert-butanol (35 ml) was added diphenylphosphonyl azide (2.6 ml), and the mixture was refluxed for 8 hours. After cooling to ambient temperature, the reaction mixture was diluted with ethyl acetate, washed in turn with hydrochloric acid (1N), aqueous sodium hydrogen carbonate, and brine, and dried over magnesium sulfate. Evaporation under reduced pressure gave a residue, which was chromatographed on silica gel (150 ml) eluting with 1-3% ethyl acetate in n-hexane to give 1-tert-butoxycarbonylamino-4-methylcyclohex-3-ene (0.82 g).
- NMR: (DMSO-d₆, δ) : 1.37 (9H, s), 1.60 (3H, s), 1.65-2.2 (6H, m), 3.2-3.4 (1H, m), 5.2-5.3 (1H, m), 6.68 (1H, br s)
- MASS: (LD) (m/z) : 234.3

### Preparation 3

To a solution of 1-tert-butoxycarbonylamino-4-methylcyclohex-3-ene (0.4 g) in a mixture of anisole (0.4 ml) and dichloromethane (0.8 ml) was added trifluoroacetic acid (1.2 ml) at 0°C and the mixture was allowed to stir at 0°C for 1 hour. Evaporation gave a residue, which was taken up into a solution of hydrogen chloride in dioxane (4N, 2 ml). Evaporation under reduced pressure and trituration with diisopropyl ether gave 1-amino-9-methylcyclohex-3-ene hydrochloride, which was used without further purification.

### Example 1 (Reference)

A solution of 1,2,3,6-tetrahydropyridine (0.25 g) and 4-phenoxycarbonylaminopyridine (0.64 g) in 1,2-dichloroethane (5 ml) was heated to 75°C for 6 hours. Evaporation of the solvent gave a residue, which was chlomatographed on silica gel (50 ml) eluting with 0-5% methanol in dichloromethane, and taken up into a solution of hydrogen chloride in ethyl acetate (4N, 2 ml). Evaporation under reduced pressure and trituration with diisopropyl ether gave 1-(pyridin-4-ylcarbamoyl)-1,2,3,6-tetrahydropyridine hydrochloride (0.43 g).
- NMR: (DMSO-d₆, δ) : 2.05-2.35 (2H, m), 3.64 (2H, t, J=6Hz), 4.05 (2H, t, J=2.5Hz), 5.6-5.8 (1H, m), 5.8-6.0 (1H, m), 8.06 (2H, d, J=7Hz), 8.55 (2H, d, J=7Hz), 10.58 (1H, s), 14.72 (1H, br s)
- MASS: (LD)(m/z) : 204.2

### Example 2 (Reference)

To a stirred solution of 1,2,3,6-tetrahydropyridine (82 mg) in tetrahydrofuran (2 ml) was added 4-fluorophenyl-isocyanate (0.112 ml) at ambient temperature. After stirring at ambient temperature for 10 hours, the solvent was removed by evaporation under reduced pressure, and the residue was triturated with diisopropyl ether to give 1-(4-fluorophenylcarbamoyl)-1,2,3,6-tetrahydropyridine (117 mg).
- NMR: (DMSO-d₆, δ) : 2.0-2.2 (2H, m), 3.51 (2H, t, J=5.7Hz), 3.85-3.95 (2H, m), 5.65-5.95 (2H, m), 6.95-7.15 (2H, m), 7.35-7.55 (2H, m), 8.47 (1H, s)
- MASS: (LD)(m/z) : 243.1

### Example 3 (Reference)

The following compound was obtained according to a similar manner to that of Example 2.

2-(4-Fluorophenylcarbamoyl)-1,2,3,4-tetrahydroisoquinoline
- NMR: (DMSO-d₆, δ) : 2.85 (2H, t, J=6Hz), 3.69 (2H, t, J=6Hz), 4.63 (2H, s), 7.07 (2H, t, J=9Hz), 7.18 (4H, s), 7.48 (2H, dd, J=5, 9Hz), 8.60 (1H, s)
- MASS: (LD)(m/z) : 293.2

### Example 4 (Reference)

To a solution of 1-tert-butoxycarbonylamino-4-methylcyclohex-3-ene (0.18 g) in a mixture of anisole (0.18 ml) and dichloromethane (0.36 ml) was added trifluoroacetic acid (0.54 ml) at 0°C and the mixture was allowed to stir at 0°C for 1 hour. Evaporation gave a residue, which was taken up into 1,2-dichloroethane (5 ml). To the mixture were added triethylamine (0.6 ml) and 4-phenoxycarbonylaminopyridine (0.183 g), and the resultant mixture was heated to 75°C for 6 hours. Evaporation gave a residue, which was chromatographed on silica gel (50 ml) eluting with 7% methanol in dichloromethane, and taken up into a solution of hydrogen chloride in ethyl acetate (4N, 2 ml). Evaporation under reduced pressure and trituration with diisopropyl ether gave N-(4-methylcyclohex-3-en-1-yl)-N'-(pyridin-4-yl)urea hydrochloride (0.144 g).
- NMR: (DMSO-d₆, δ) : 1.64 (3H, s), 1.4-2.4 (6H, m), 3.6-3.9 (1H, m), 5.2-5.35 (1H, m), 7.26 (1H, d, J=8Hz), 7.82 (2H, d, J=7Hz), 8.51 (2H, d, J=7Hz), 10.91 (1H, s), 14.50 (1H, br s)
- MASS: (LD)(m/z) : 232.2

### Example 5 (Reference)

To a suspension of 1-amino-4-methylcyclohex-3-ene hydrochloride (0.103 g) in dichloromethane (5 ml) were added in turn pyridine (0.14 ml) and 4-fluorobenzoyl chloride (83 µl) at 0°C. The mixture was allowed to warm to ambient temperature and stirred for 1 hour, which was taken up into a mixture of water and ethyl acetate. The separated organic layer was washed in turn with hydrochloric acid (1N), aqueous sodium hydrogen carbonate, and brine, and dried over magnesium sulfate. Evaporation under reduced pressure gave a residue, which was chromatographed on silica gel (50 ml) eluting with 0-20% ethyl acetate in n-hexane to give 1-(4-fluorobenzoylamino)-4-methylcyclohex-3-ene (98 mg).
- NMR: (DMSO-d₆, δ) : 1.59 (3H, s), 1.4-2.3 (6H, m), 3.8-4.1 (1H, m), 5.35-5.5 (1H, m), 7.27 (2H, t, J=9Hz), 7.89 (2H, dd, J=5, 9Hz), 8.25 (1H, d, J=7Hz)
- MASS: (APCI)(m/z) : 234

### Example 6

The following compound was obtained according to a similar manner to that of Example 5.

2-(4-Fluorobenzoylamino)-1,2,3,4-tetrahydronaphthalene
- NMR: (DMSO-d₆, δ) : 1.65-1.9 (1H, m), 1.95-2.25 (1H, m), 2.7-3.1 (4H, m), 4.05-4.3 (1H, m), 7.08 (4H, s), 7.2-7.4 (2H, m), 7.85-8.05 (2H, m), 8.45 (1H, d, J=7.5Hz)
- MASS: (APCI)(m/z) : 270

### Example 7 (Reference)

To a suspension of 1-amino-4-methylcyclohex-3-ene hydrochloride (103 mg) in dichloromethane (5 ml) were added in turn pyridine (0.14 ml), 4-pyridinecarbonyl chloride hydrochloride (0.124 g) and N,N-dimethylaminopyridine (0.11 g) at 0°C. The mixture was allowed to warm to ambient temperature and was allowed to stir for 1 hour. The reaction mixture was taken up into a mixture of water and ethyl acetate, and adjusted pH to 4.6. The separated organic layer was washed in turn with water and brine, and dried over magnesium sulfate. Evaporation under reduced pressure gave a residue, which was triturated with diisopropyl ether to give 1-(pyridin-4-ylcarbonylamino)-4-methylcyclohex-3-ene (46 mg) .
- NMR: (DMSO-d₆, δ) : 1.64 (3H, s), 1.45-3.35 (6H, m), 3.8-4.1 (1H, m), 5.25-5.45 (1H, m), 7.74 (2H, dd, J=1.6, 4.5Hz), 8.53 (1H, d, J=7.5Hz), 8.70 (2H, dd, J=1.6, 4.5Hz)
- MASS: (APCI)(m/z) : 217

### Example 8 (Reference)

The following compound was obtained according to a similar manner to that of Example 7.

2-(Pyridin-4-ylcarbonylamino)-1,2,3,4-tetrahydronaphthalene
- NMR: (DMSO-d₆, δ) : 1.65-1.9 (1H, m), 1.95-2.15 (1H, m), 2.7-3.15 (4H, m), 4.05-4.3 (1H, m), 7.10 (4H, s), 7.78 (2H, dd, J=1.6, 4.5Hz), 8.65-8.8 (3H, m)
- MASS: (APCI)(m/z) : 253

### Example 9 (Reference)

1) To a solution of 1-tert-butoxycarbonylamino-4-methylcyclohex-3-ene (0.18 g) in a mixture of anisole (0.18 ml) and dichloromethane (0.36 ml) was added trifluoroacetic acid (0.54 ml) at 0°C and the mixture was allowed to stir at 0°C for 1 hour. Evaporation gave a residue containing 1-amino-4-methylcyclohex-3-ene.
2) The residue containing 1-amino-4-methylcyclohex-3-ene was taken up into dichloromethane (5 ml). To the mixture were added triethylamine (0.6 ml) and 4-fluorophenylisocyanate (97 pl) at 0°C and the resultant mixture was allowed to stir for 30 minutes at 0°C. Evaporation under reduced pressure gave a residue, which was taken up into a mixture of water and ethyl acetate. The separated organic layer was washed with brine, evaporated under reduced pressure, and triturated with n-hexane to give N-(4-methylcyclohex-3-en-1-yl)-N'-(4-fluorophenyl)urea (0.206 g).
   - NMR: (DMSO-d₆, δ) : 1.63 (3H, s), 1.3-1.9 (3H, m), 1.9-2.1 (2H, m), 2.1-2.4 (1H, m), 3.6-3.85 (1H, m), 5.25-5.35 (1H, m), 6.07 (1H, d, J=8Hz), 7.04 (2H, t, J=9Hz),7.36 (2H, dd, J=5, 9Hz), 8.38 (1H, s)
   - MASS: (LD)(m/z) : 271.2

### Example 10 (Reference)

The following compound was obtained by using 2-amino-1,2,3,4-tetrahydronaphthalene as a starting compound according to a similar manner to that of Example 2.

N-(4-Fluorophenyl)-N'-(1,2,3,4-tetrahydronaphthalen-2-yl)urea
- NMR: (DMSO-d₆, δ) : 1.6-1.8 (1H, m), 1.8-2.05 (1H, m), 2.63 (1H, dd, J=8, 16Hz), 2.83 (2H, t, J=7Hz), 3.02 (1H, dd, J=5, 16Hz), 3.8-4.1 (1H, m), 6.22 (1H, d, J=7.5Hz), 6.95-7.2 (2H, m), 7.12 (4H, s), 7.3-7.45 (2H, m), 8.40 (1H, s)
- MASS: (APCI)(m/z) : 285

### Example 11 (Reference)

To a solution of aminodiphenylmethane (0.4 g) in dichloromethane (5 ml) were added in turn pyridine (0.21 ml) and 4-fluorobenzoyl chloride (0.23 ml) at 0°C. The mixture was allowed to warm to ambient temperature and stirred for 1 hour, which was taken up into a mixture of water and ethyl acetate. The separated organic layer was washed in turn with hydrochloric acid (1N), aqueous sodium hydrogen carbonate and brine, and dried over magnesium sulfate. Evaporation under reduced pressure gave a residue, which was triturated with diisopropyl ether to give (4-fluorobenzoylamino)-diphenylmethane (0.49 g).
- NMR: (DMSO-d₆, δ): 6.40 (1H, d, J=9Hz), 7.2-7.45 (12H, m), 8.01 (2H, dd, J=5, 9Hz), 9.30 (1H, d, J=9Hz)
- MASS: (APCI) (m/z): 306

### Example 12 (Reference)

To a solution of 4-fluoroaniline (0.2 g) in . dichloromethane (10 ml) were added in turn pyridine (0.19 ml) and diphenylcarbamoyl chloride (0.417 g) at 0°C. The mixture was allowed to warm to ambient temperature and stirred for 10 hours, and to the mixture was added N,N-dimethylaminopyridine (0.22 g), and the mixture was allowed to stir for another 1 hour. The reaction mixture was taken up into a mixture of water and ethyl acetate. The separated organic layer was washed in turn with hydrochloric acid (1N), aqueous sodium hydrogen carbonate and brine, and dried over magnesium sulfate. Evaporation under reduced pressure gave a residue, which was triturated with diisopropyl ether to give N,N-diphenyl-N'-4-fluorophenylurea (0.384 g).
- NMR: (DMSO-d₆, δ): 7.07 (2H, t, J=9Hz), 7.15-7.3 (6H, m), 7.3-7.5 (6H, m), 8.45 (1H, s)
- MASS: (APCI) (m/z): 307

### Example 13

To a solution of (R)-1,2,3,4-tetrahydronaphthalen-2-ylamine hydrochloride (0.9 g) in dichloromethane (15 ml) were added in turn triethylamine (1.71 ml) and 4-fluorobenzoyl chloride (0.58 ml) at 0°C. The mixture was allowed to warm to ambient temperature and stirred for 1 hour, which was taken up into a mixture of water and ethyl acetate. The separated organic layer was washed in turn with hydrochloric acid (1N), aqueous sodium hydrogen carbonate and brine, and dried over magnesium sulfate. Evaporation under reduced pressure gave a residue, which was triturated with diisopropyl ether to give (R)-4-fluoro-N-(1,2,3,4-tetrahydronaphthalen-2-yl)benzamide (1.26 g).
- NMR: (DMSO-d₆, δ): 1.60-1.89 (1H, m), 1.95-2.16 (1H, m), 2.70-3.14 (4H, m), 4.05-4.30 (1H, m), 7.09 (4H, s), 7.30 (2H, t, J=8.9Hz), 7.86-8.04 (2H, m), 8.45 (1H, d, J=7.6Hz)
- MASS: (APCI) (m/z): 270.3

### Example 14

To a solution of (S)-1,2,3,4-tetrahydronaphthalen-2-ylamine hydrochloride (0.9 g) in dichloromethane (15 ml) were added in turn triethylamine (1.71 ml) and 4-fluorobenzoyl chloride (0.58 ml) at 0°C. The mixture was allowed to warm to ambient temperature and stirred for 1 hour, which was taken up into a mixture of water and ethyl acetate. The separated organic layer was washed in turn with hydrochloric acid (1N), aqueous sodium hydrogen carbonate and brine, and dried over magnesium sulfate. Evaporation under reduced pressure gave a residue, which was triturated with diisopropyl ether to give (S)-4-fluoro-N-(1,2,3,4-tetrahydronaphthalen-2-yl)benzamide (1.26 g).
- NMR: (DMSO-d₆, δ): 1.60-1.89 (1H, m), 1.95-2.16 (1H, m), 2.70-3.14 (4H, m), 4.05-4.30 (1H, m), 7.09 (4H, s), 7.30 (2H, t, J=8.9Hz), 7.86-8.04 (2H, m), 8.45 (1H, d, J=7.6Hz)
- MASS: (APCI) (m/z): 270.3

### Example 15

To a solution of 7-methoxy-1,2,3,4-tetrahydronaphthalen-2-ylamine (0.49 g) in dichloromethane (5 ml) were added in turn pyridine (0.29 ml) and 4-fluorobenzoyl chloride (0.33 ml) at 0°C. The mixture was allowed to warm to ambient temperature and stirred for 1 hour, which was taken up into a mixture of water and ethyl acetate. The separated organic layer was washed in turn with hydrochloric acid (1N), aqueous sodium hydrogen carbonate and brine, and dried over magnesium sulfate. Evaporation under reduced pressure gave a residue, which was triturated with diisopropyl ether to give 4-fluoro-N-(7-methoxy-1,2,3,4-tetrahydronaphthalene-2-yl)-benzamide (497 mg).
- NMR: (DMSO-d₆, δ): 1.60-1.85 (1H, m), 1.92-2.13 (1H, m), 2.63-3.10 (4H, m), 3.70 (3H, s), 4.00-4.25 (1H, m), 6.60-6.79 (2H, m), 7.00 (1H, d, J=8.2Hz), 7.30 (2H, t, J=8.9Hz), 7.89-8.04 (2H, m), 8.44 (1H, d, J=7.6Hz)
- MASS: (APCI) (m/z): 300

### Example 16

To a solution of 6-methoxy-1,2,3,4-tetrahydronaphthalen-2-ylamine (0.57 g) in dichloromethane (5 ml) were added in turn triethylamine (0.46 ml) and 4-fluorobenzoyl chloride (0.30 ml) at 0°C. The mixture was allowed to warm to ambient temperature and stirred for 1 hour, which was taken up into a mixture of water and ethyl acetate. The separated organic layer was washed in turn with hydrochloric acid (1N), aqueous sodium hydrogen carbonate and brine, and dried over magnesium sulfate. Evaporation under reduced pressure gave a residue, which was triturated with diisopropyl ether to give 4-fluoro-N-(6-methoxy-1,2,3,4-tetrahydronaphthalen-2-yl)-benzamide (0.59 g).
- NMR: (DMSO-d₆, δ): 1.60-1.85. (1H, m), 1.92-2.10 (1H, m), 2.60-3.07 (4H, m), 3.71 (3H, s), 4.00-4.30 (1H, m), 6.60-6.75 (2H, m), 6.99 (1H, d, J=8.2Hz), 7.30 (2H, t, J=8.9Hz), 7.80-8.04 (2H, m), 8.42 (1H, d, J=7.6Hz)
- MASS: (APCI) (m/z): 300

### Example 17

To a solution of indan-2-ylamine (0.297 g) in dichloromethane (5 ml) were added in turn pyridine (0.23 ml) and 4-fluorobenzoyl chloride (0.26 ml) at 0°C. The mixture was allowed to warm to ambient temperature and stirred for 1 hour, which was taken up into a mixture of water and ethyl acetate. The separated organic layer was washed in turn with hydrochloric acid (1N), aqueous sodium hydrogen carbonate and brine, and dried over magnesium sulfate. Evaporation under reduced pressure gave a residue, which was triturated with diisopropyl ether to give 4-fluoro-N-(indan-2-yl)benzamide (0.325 g).
- NMR: (DMSO-d₆, δ): 2.94 (2H, dd, J=6.7, 16.0Hz), 3.24 (2H, dd, J=6.7, 16.0Hz), 4.55-4.80 (1H, m), 7.06-7.40 (6H, m), 7.83-8.04 (2H, m), 8.67 (1H, d, J=6.7Hz)
- MASS: (APCI) (m/z): 256

## Claims

1. A compound of the formula (I): 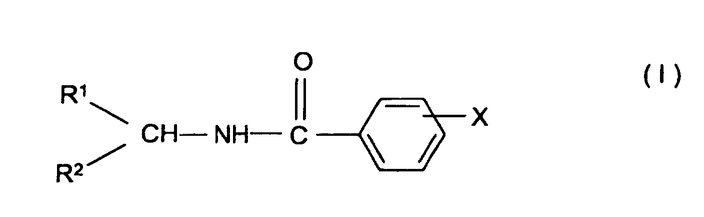 wherein R¹ and R² are taken together to form pentenylene condensed with benzene optionally substituted with (C₁-C₆) alkyl, (C₁-C₆) alkoxy, phenyl or halogen, and
X is halogen,
or,
R¹ and R² are taken together to form butenylene condensed with benzene, and
X is halogen,
and the indan ring formed by taking together R¹, R² and CH is substituted by -NH-CO-(substituted) phenyl at the 2-position,
and its salt.

2. A compound according to claim 1, wherein R¹ and R² are taken together to form butenylene condensed with benzene.

3. A compound according to Claim 2, which is 4-fluoro-N-(indan-2-yl)benzamide.

4. A compound according to claim 1, wherein
R¹ and R² are taken together to form pentenylene which is condensed with benzene.

5. A compound according to claim 4, which is (R)-4-fluoro-N-(1,2,3,4-tetrahydronaphthalen-2-yl)benzamide.

6. A process for preparing a compound of the formula: 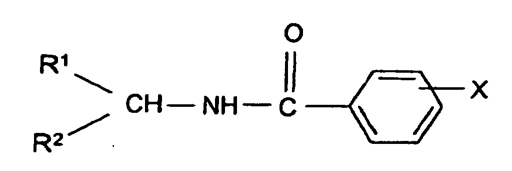 wherein R¹, R², and X are each as defined In claim 1.
or is salt, which comprises,
reacting a compound of the formula: 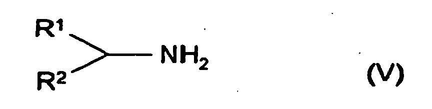 or its salt with a compound of the formula: 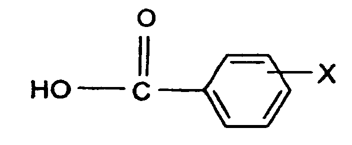 or its reactive derivative at the carboxy group or a salt thereof to provide a compound of the formula: 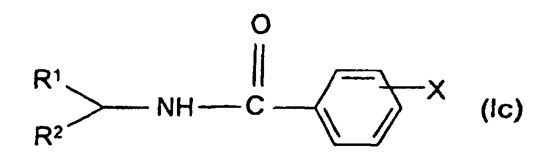 or its salt,
in the above formulas R¹, R² end X are each as defined in claim 1.

7. A pharmaceutical composition comprising a compound of any of claims 1 to 5, as an active ingredient, in association with a pharmaceutically, acceptable substantially non-toxic carrier or excipient.

8. A compound of any of claims 1 to 5 for use as a medicament.

9. Use of the compound as defined in any of claims 1 to 5 for the manufacture of a medicament for treating and /or preventing amnesia, schizophrenia or dementia in mammals.

## Patentansprüche

1. Verbindung der Formel (I): 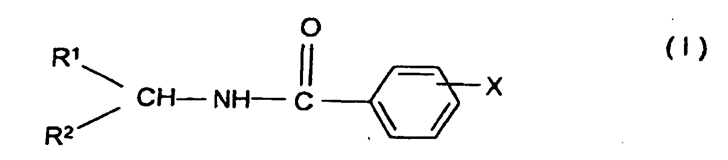 wobei R¹ und R² zusammengenommen Pentenylen ergeben, welches mit Benzol kondensiert ist, das gegebenenfalls mit (C₁-C₆) Alkyl, (C₁-C₆) Alkoxy, Phenyl oder Halogen substituiert ist, und
X Halogen ist, oder
R¹ und R² gemeinsam Butenylen bilden, welches mit Benzol kondensiert ist, und
X Halogen ist,
und der durch Zusammennehmen von R¹, R² und CH gebildete Indanring durch -NH-CO-(substituiertes) Phenyl in 2- Position substituiert ist,
und ihr Salz.

2. Verbindung nach Anspruch 1, wobei R¹ und R² zusammengenommen mit Benzol kondensiertes Butenylen bilden.

3. Verbindung nach Anspruch 2, die 4-Fluoro-N-(indan-2-yl)benzamid ist.

4. Verbindung nach Anspruch 1, wobei R¹ und R² zusammengenommen mit Benzol kondensiertes Pentylen bilden.

5. Verbindung nach Anspruch 4, die (R)-4-Fluoro-N-(1,2,3,4-tetrahydronaphthalin-2-yl)benzamid ist.

6. Verfahren zur Herstellung einer Verbindung der Formel: 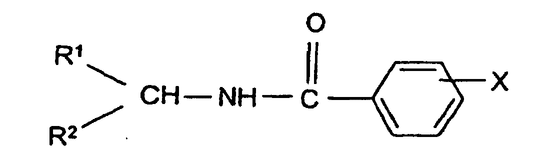 wobei R¹, R² und X jeweils wie in Anspruch 1 definiert sind,
oder von deren Salz, weiches umfasst
Umsetzen einer Verbindung der Formel: 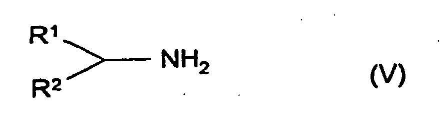 oder von deren Salz mit einer Verbindung der folgenden Formel: 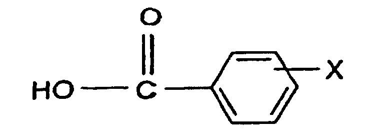 oder ihrem reaktiven Derivat an der Carboxygruppe oder einem Salz davon zur Herstellung einer Verbindung der Formel 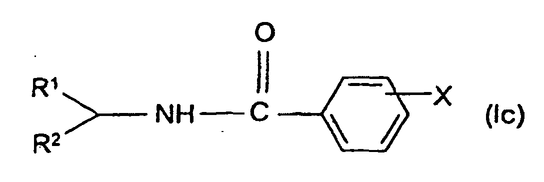 oder von deren Salz,
wobei in den obigen Formeln R¹, R² und X jeweils wie in Anspruch 1 definiert sind.

7. Pharmazeutische Zusammensetzung, umfassend eine Verbindung nach einem der Ansprüche 1 bis 5 als aktiven Bestandteil in Verbindung mit einem pharmazeutisch verträglichen, im wesentlichen nicht-toxischen Träger oder Excepienten.

8. Verbindung nach einem der Ansprüche 1 bis 5 zur Verwendung als Medikament.

9. Verwendung einer Verbindung, wie in einem der Ansprüche 1 bis 5 zur Herstellung eines Medikaments zur Behandlung und/oder Vorbeugung von Amnesie, Schizophrenie oder Demenz bei Säugern.

## Revendications

1. Composé de formule (I) : 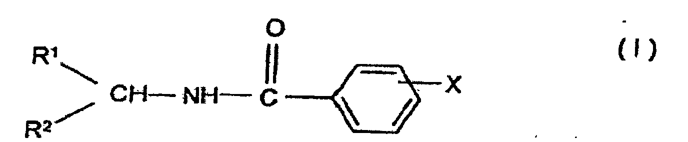 dans laquelle R¹ et R² sont pris conjointement pour former un penténylène condensé à un benzène éventuellement substitué par un groupe alkyle (en C₁-C₆), alcoxy (en C₁-C₆), phényle ou un atome d'halogène, et
X est un atome d'halogène,
ou,
R¹ et R² sont pris conjointement pour former un buténylène condensé à un benzène, et
X est un atome d'halogène,
et le noyau indane formé par prise conjointe de R¹, R² et CH est substitué par un groupe phényle -NH-CO-substitué en position 2,
ou son sel.

2. Composé selon la revendication 1, dans lequel R¹ et R² sont pris conjointement pour former un buténylène condensé à un benzène.

3. Composé selon la revendication 1, qui est le 4-fluoro-N-(indan-2-yl)benzamide.

4. Composé selon la revendication 1, dans lequel R¹ et R² sont pris conjointement pour former un penténylène condensé à un benzène.

5. Composé selon la revendication 4, qui est le (R)-4-fluoro-N-(1,2,3,4-tétrahydronaphtalén-2-yl)benzamide.

6. Procédé de préparation d'un composé de formule : 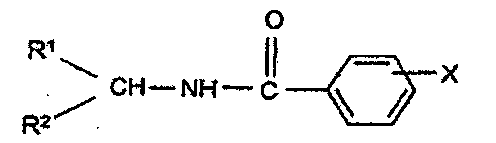 dans laquelle R¹, R² et X sont chacun tels que définis dans la revendication 1,
ou son sel, qui comprend :
la réaction d'un composé de formule : 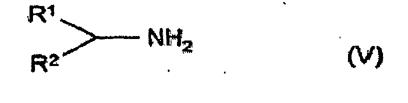 ou son sel avec un composé de formule : 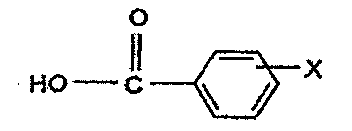 ou son dérivé réactif, au niveau du groupe carboxy ou un sel de celui-ci pour donner un composé de formule : 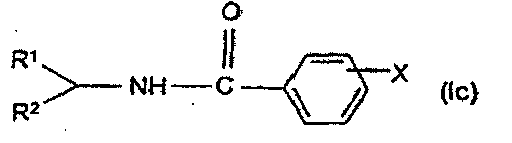 ou son sel,
dans les formules ci-dessus, R¹, R² et X sont chacun tels que définis dans la revendication 1.

7. Composition pharmaceutique comprenant un composé selon l'une quelconque des revendications 1 à 5, en tant qu'ingrédient actif, en association avec un véhicule ou un excipient pharmaceutiquement acceptable, essentiellement non toxique.

8. Composé selon l'une quelconque des revendications 1 à 5, à utiliser comme médicament.

9. Utilisation d'un composé selon l'une quelconque des revendications 1 à 5, pour la fabrication d'un médicament pour traiter et/ou prévenir l'amnésie, la schizophrénie ou la démence chez des mammifères.
